Europäisches Patentamt

European Patent Office

Office européen des brevets

(11) Publication number: **0 257 507**
**A1**

(12) **EUROPEAN PATENT APPLICATION**

(21) Application number: 87111890.7

(22) Date of filing: 17.08.87

(51) Int. Cl.⁴ **C12N 15/00** , C12P 21/00 , C12N 5/00 , A61K 39/29 , G01N 33/576

The applicant has filed a statement in accordance with Rule 28 (4) EPC (issue of a sample only to an expert). Accession number(s) of the deposit(s): CNCM I-574 and I-575.

(30) Priority: 17.08.86 IL 79740

(43) Date of publication of application: 02.03.88 Bulletin 88/09

(84) Designated Contracting States: AT BE CH DE ES FR GB GR IT LI LU NL SE

(71) Applicant: **YEDA RESEARCH AND DEVELOPMENT LTD.**
**P.O. Box 95**
**Rehovot(IL)**

(72) Inventor: **Shaul, Yosef**
**Mercaz Shapira**
**79411 Israel(IL)**

(74) Representative: **Vossius & Partner**
**Siebertstrasse 4 P.O. Box 86 07 67**
**D-8000 München 86(DE)**

(54) **Hepatitis vaccine.**

(57) The invention provides a vaccine against Hepatitis B virus (HBV) produced by the technique of genetic engineering. A DNA clone designated as clone Al26, produced significant amounts of HBsAg, and the sequence of the relevant portions thereof was determined. Two CHO cell lines into which this clone was inserted, have been deposited at the Institute Pasteur under Numbers I574 and I575. The invention relates to cell lines for producing HBsAg; the production of HBsAg by means of such cell lines and to vaccines against Hepatitis B based on the thus obtained HBsAg.

EP 0 257 507 A1

## HEPATITIS VACCINE

### Background of the invention:

Hepatitis B is a very wide-spread disease and in certain countries carriers of the disease constitute about 10% of the population. In such countries Hepatitis B and liver cirrhosis, as well as hepatocellular carcinoma are amongst the major causes of mortality. There exists strong evidence that Hepatitis B virus (HBV) causes hepatocellular carcinoma (HCC). In view of the above it is clear that there exists an urgent need for a safe and economically feasible vaccine. The main problem is that HBV cannot be propagated in tissue cultures. Several attempts have been made to produce vaccines by the techniques of genetic engineering, see Tiollais et al: The Hepatitis B virus, Nature 317 489 (1985).

The immunological markers characteristic of the infection of Hepatitis B virus (HBV) include three coded proteins: surface antigen HBsAg; core antigen HBcAg; and core derived antigen HBeAg. Two main classes of particles are found in the plasma of viremic HBV carriers: 22 nm particles and 42 nm particles known as Dane particles, the latter is composed of an outer surface antigen envelope which surrounds a core particle (genomic HBV DNA, with core antigen and DNA polymerase). The 22 nm particles are produced in chronic carriers of HBV. They are also produced by hepatomas associated with HBV. Since HBV surface antigen specific antibodies are protective against HBV infection the 22 nm particles which are highly immunogenic have served as a source for an HBV active vaccine. The 22 nm particles isolated from viremic carriers are composed of several related proteins. The major protein is P24; its glycosylated form is known as GP27. The minor protein is P33; its glycosylated form is known as GP36. Dane particles contain a third minor protein, P39, and its glycosylated form, GP42. P24 which has been synthesized in yeast and bacterial systems was expected to serve as a suitable vaccine against HBV. However, it was recently shown that the longer proteins namely P33 and GP36 are far more immunogenic than P24 and GP27.

### Production of the HBsAg Vaccine in CHO cells:

### Transcription of Hepatitis Surface Antigen Gene in Eucaryotic Cells

The sequence of the open reading frame of HBsAg gene may be divided into three independently expressed parts: I) pre-SI, present only in the P39 and GP42, 2) pre-S2, present in the GP33 and GP36 and 3) and S, present in all the proteins. The transcription of the HBsAg gene is regulated by at least two independent promoters. The longest transcript is achieved by activation of the "TATA" box promoter at the 5′ end of the gene. This transcript contains the entire information for synthesis of all the HBsAg proteins. The HBsAg "TATA" box promoter is almost inactive when the gene is stably introduced into culture cells. However, low activity of this promoter was detected in Alexander cells, a human hepatoma cell line known to produce the 22 nm paricles. A smaller transcript of HBsAg gene is synthesized by another promoter, named "SV40 like" promoter. This transcript does not contain the pre-SI region and therefore does not encode for P39 and GP42. Because the "SV40 like" promoter has multiple start sites it produces two classes of RNA which may code for either P33 and GP36 or P24 and GP27. The production of an HBV vaccine in tissue culture has several advantages:

I. Unlike bacterial systems the tissue culture system produces both the glycosylated and non-glycosylated HBsAg forms.

2. Only in tissue culture the complexity of the entire HBV surface gene can be expressed. Namely, use of the HBV authentic promoters results in the production of transcript coding for all three HbsAg related proteins and their related glycosylated forms.

3. In tissue culture HBsAg is assembled into stable 22 nm particles which are far more immunogenic than soluble HBsAg produced in other heterologous systems.

4. Unlike bacteria or yeast, mammalian cells produce and secrete 22 nm particles. This simplifies the purification of the vaccine.

## Current Available Tissue Culture Systems

A major problem in the synthesis of HBsAg in tissue culture is obtaining the complete spectrum of the HBsAg proteins in a given culture. Efforts to introduce HBV sequences into cell culture and to produce all the proteins have failed. However, use of heterologous oncogenic viral expression vectors to direct the expression of HBsAg, resulted in relatively large quantities of HBsAg proteins. This approach of mass preparations of HBsAg suffers from several disadvantages: l) Direct usage of genetic elements from other organic viruses which will undoubtedly cause regulatory problems; 2) The resulting HBsAg products did not contain the P39 and GP42 proteins because the viral transcription regulation elements were inserted within the pre-Sl region; 3) The usage of heterologous regulatory element for expression of the different HBsAg protein distorts stochiometric amounts of these products resulting in inefficient assembly of 22 nm like particles. A. Freytag et al., (EMBO J. 4:249-255 (l985)) describe the espression in mouse Tk⁻ cells of an HBSAg gene isolated from Alexander cells. However, the HBV sequence they use is terminated at the 5' end by a Hind III site. Hence they are lacking HBV and host sequences upstream of this Hind III site (Fig. I, clones l0.7 and l0.5) Michael et al (PNAS 8l:7708-l2 (l984)) describe the expression in CHO cells of an S gene isolated from a prototype HBV genome placed under the control of an SV40 promoter or a MMTV-LTR promoter. There is no disclosure of an HBSAg gene isolated from Alexanderr Cells nor is there a disclosure of expression of HBSAg from the HBV promoters.

The present invention uses an S gene isolated from Alexander cells-a cell line which constitutively overexpresses the HBSAg. In one embodiment of the invention the Alexander cell line derived gene directs the expression of a novel HBSAg polypeptide. The present invention makes use of an S gene complex comprising the TATA and SV40 like promoters and the pre-Sl, pre-S2 and S coding regions.

According to the present invention, in order to overcome the problems set out above, and in order to produce large quantities of HBsAg which can be used as active ingredient in vaccines against HBV, the HBsAg transcriptionally active gene from Alexander cells was isolated and used in the synthesis of HBsAg. This choice was based on the consideration that as Alexander cells produce relatively large amounts of immunogenic HBsAg as 22 nm particles, this cell line should contain a gene which is HBsAg active which, when isolated, can be introduced into non-human cell lines. Analysis of HBsAg mRNA demonstrated that both promoters of the HBsAg gene are active, implying that the gene is intact and capable of synthesizing the entire spectrum of HBsAg proteins. It is evident that Alexander cells, which are human hepatoma cells, cannot be used for vaccine production.

## Summary of the invention:

There are provided novel vaccines against Hepatits B virus (HBV). These are produced by techniques of genetic engineering , and result in comparatively high yields of active material.

Cell line CHO²⁰⁰ᴹʳ p7.5Al26 and CHO⁵⁰ᴹʳ p7.5Al26 have been deposited with the Institute Pasteur under Deposition nos. I-574 and I-575 respectively.

## Detailed Description of the Invention:

### Isolation of an Intact and Active HBsAg Gene from Alexander Cell Line

The integrated HBV DNA in Alexander cells was isolated by molecular cloning and was characterized by restriction enzyme mapping and partial DNA sequence analysis (Shaul, Y. et al., J. Virol. 5l, 776-787 (l984)).

Seven distinct integrated HBV sequences were defined. The seven genes seem to represent the entire complement of integrated HBV sequences. In order to isolate a clone which expresses the HBsAg gene, each of the seven clones was inserted into CHO dhfr⁻ cell line using the dhfr gene as a selective marker using the calcium phosphate coprecipitate technique (Graham & Van der Eb, Virology 52, 456-467 (l973)).

Mass cultures, composed of l0-20 colonies of cells that acquired both the dhfr gene and probably the HBV DNA from Alexander cells were tested for synthesis and secretion of HBsAg by the radioimmunoassay technique using a diagnostic Abbott Kit (AUSRIA II, Abbott). Only one clone, Al26 produced significant amounts of HBsAg. The entire sequence of HBV portion of Al26 was determined and the following conclusions emerged: I) the HBV sequence is related but not identical to published HBV sequence of subtype adw₂ Fig. I). 2) The entire coding frames of pre-Sl, pre-S2 and S region are present including all initiation ATG's and translation termination codons, without any frameshift mutations, deletions or insertions. 3) The HBsAg gene in AI26 contains the "TATA" box promoter, the "SV40 like" promoter, the HBV enhancer element and 2-3 polyadenylation sites of the those sequences downstream from the 3' end of the HBV sequence (Fig. I). 4) The entire core gene is not present in AI26 and therefore infection virus cannot be produced. The structure of AI26 is depicted in Fig. I. This DNA sequence of the pre-S2 region, in which the amino acid sequence of the predicted protein differs from all previously published sequences is shown. Also shown in this figure, the novel HBV sequences found in the 3' region of the S gene of AI26.

## High Production of HBsAg by Subclone or Clone 9H

Although AI26(about I4Kb Alexander DNA insert in Charon 28 phage) expressed and produced small amounts of HBsAg in CHO cells, the amount was not satisfactory for large scale preparation of this protein. We believe that the excess of non-relevant host and phage DNA may have an inhibitory effect on HBsAg gene expression.

Therefore, we subcloned a 7.5 Kb Eco RI fragment containing the entire integrated HBV sequence and 2 Kb host sequence at the 5' and 3 Kb host sequence at the 3' end of the HBV sequence in an Eco RI side of pBR322 plasmid.

This new plasmid, called p7.5AI26 (Fig. I), was inserted into CHO$^{dhfr-}$cells. The ratio of p7.5AI26 DNA to plasmids containing the dhfr gene was I0:I in the transfection mixture; this procedure allows multiple insertion of p7.5AI26 in any selected colony. Three plates, each containing a pool of 4-5 clones of dhfr positive cells were tested for HBsAg expression by AUSRIA II Abbott RIA Kit. Two plates expressed high levels of HBsAg which were estimated to be 8-I0 fold higher than the amount of HBsAg obtained by CHO cells transfected with AI26.

It is known that treatment of cells with methotrexate (MTX an inhibitor of dhfr) results in amplification of the dhfr gene in the survivor colonies, which also results in higher expression of the gene inserted by the cotransfection procedure. We treated the selected colonies with 50 to 200 nM MTX for three weeks and found that the resistant cells (CHO$^{50M_r}$p7.5 AI26 and CHO$^{200M_r}$p7.5 AI26) produced I0 -fold more HBsAg than the parental colonies.

## Expression Levels of HBsAg by the CHO $^{M_r}$p7.5AI26 Cell Line

The constitutive expression of immunoreactive HBsAg by this cell line was initially determined assaying the medium of confluent cultures. Using the Abbott RIA HBsAg kit it was determined that the medium of confluent CHO$^{M_r}$p7.5AI26 cultures 3-5 mg/L immunoreactive HBsAg. In order to evaluate the exact amount of HBsAg produced by this cell line during exponentially growing cells we had plated different numbers of cells and the medium was changed daily and were assayed for HBsAg using the Abbott RIA kit (Table I).

The following properties of the system were assayed:

I. The HBsAg secreted by the CHO$^{M_r}$p7.5AI26 cells is stable for many months when stored either at 4°C or at -20°C.

2. To date we have passaged the cells over 50 times without any reduction in HBsAg secretion.

3. Antibiotic (Penicillin, Streptomycin and Garamycin) as well as antifungal (Fungizon) agents had no effect on cell growth or HBsAg expression.

4. CHO$^{M_r}$p7.5AI26 cells were viable and produced HBsAg after freezing (I0% Glycerol) for a few months in liquid nitrogen.

5. The cells were grown in Gibco's Alpha medium supplemented with I0% dialyzed FCS. However, the cells can be grown in regular DMEM supplemented with proline and I0% dialysed and FCS.

6. The methortexate (50 nM) was present during all cell passages. However, it may be deleted from the culture medium at least for several passages without reducing expression of HBsAg.

Properties of HBsAg Synthesized by the CHO$^{Mr}$p7.5Al26 Cells

To further characterize the HBsAg produced and secreted by this cell line, HBsAg from these cells and from control Alexander cells was partially purified.

350 ml medium from confluent cultures was precipitated with 45% saturated ice cold ammonium sulphate. This procedure removes most of the albumin present in the culture medium. The pellet was dissolved in l5 ml TS (Tris 7.4/saline) and dialyzed extensively against TS buffer. Over 90% of the HBsAg, as determined by the Abbott HBsAg RIA test, was recovered by this stage. It was estimated that the CHO$^{50Mr}$p7.5Al26 cell produce 20-fold (2 mg/350 ml medium) more HBsAg than the control Alexander cells.

An aliquot of the partially purified HBsAg pellet was applied to a CsCl buoyant density gradient. The HBsAg banded at a density of l.22 g/ml identical to HbsAg from serum of carrier patients. The HBsAg from the gradient was analyzed by electro microscopy using the negative staining procedure. The majority of the HBsAg appears by EM as spherical 22 nm particles. The size and form of these particles appear identical to particles isolated from human serum (Gerin et. al., J. Virol. 7, 569-570, (l97l)) or from the control Alexander cells.

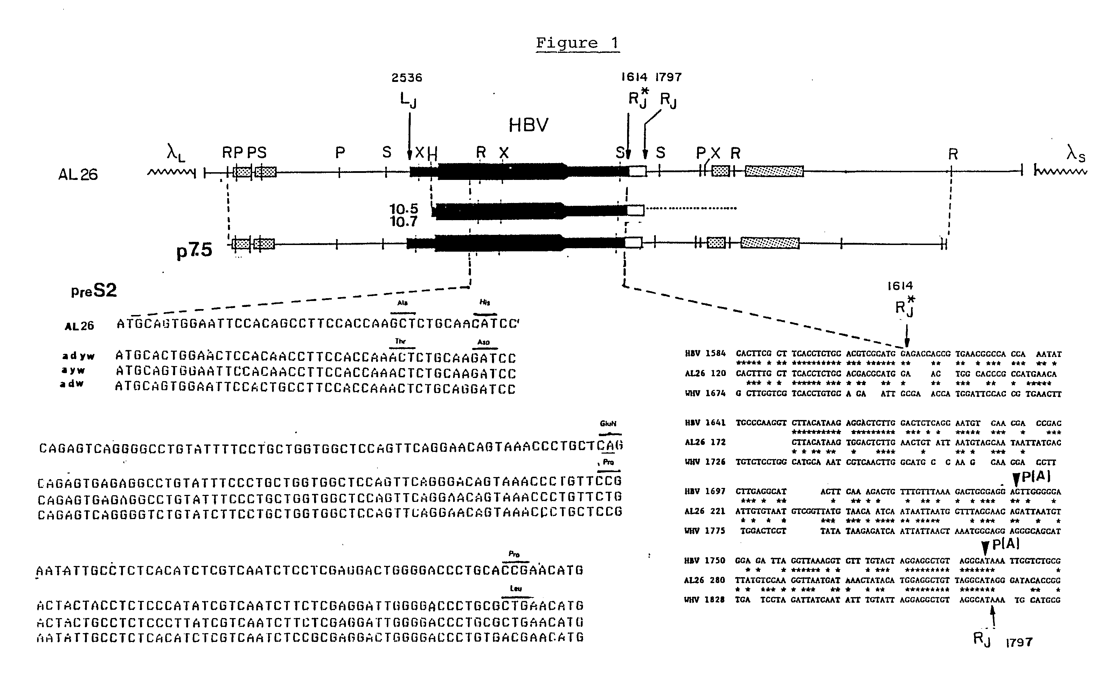

Legend to the Figure:

Fig. l: The structure and partial sequence of the integrated HBV DNA in Al26.

The restriction enzyme map of Al26 is shown. The enzymes that were used are EcoRI(R), Pst I(P) Sma I(S), Hind III(H) and Xba I (X). The dotted boxes represent the Alu I repeat units. L$_J$ indicates the left end of the integrated HBV DNA and the R$_J$ the right rnf, this end was previously mapped incorrectly at position l6l (R$_J^*$). The HBV portion in clones l0.76 and l0.5 (Hofschneider et al) are also shown dith different host sequence (dotted line). The portion of Al26 that was cloned in pBR322 to construct the 07.5 is shown. The DNA sequence of the pre-S2 of Al26 is compared to that of those indicated HBV subtypes (adyw, ayw and adw). This portion of HBV sequence contains the following four unique amino acid substitutions which are not present in other vital subtypes; Alu instead of Thr, His instead of Asp, GluN instead of Pro and Pro instead of Leu. At the right side of the figure we compared the sequence of Al26 in the region previously believed to be of host origin (between R$_J^*$ to R$_J$) to that of HBV and Woodstuck hepatitis virus (WHV). This comparison shown that this portion of Al26 contains a novel HBV DNA with relatively low homology to the published cital sequence. The region contains also the polyadenylation site (PA) of the vital mRNA which initiates at the S gene promoter.

Table 1:

The level of HBsAg productio by CHO$^{200Mr}$ p7.5Al26.

|  | HBsAg production ng/day/in 4 ml culture | | | |
|---|---|---|---|---|
| Days after plating | 1 | 2 | 3 | total ng |
| Number of plated cells | | | | |
| 10$^5$ | 320 | 340 | 500 | 1160 |
| 2.5 x 10$^5$ | 530 | 570 | 700 | 1800 |
| 5 x 10$^5$ | 800 | 850 | 900 | 2550 |

Cells were plated in 40 mm dish in 4 ml medium (αmedium supplemented with l0% dialized FCS and 200 nM of methotrexate). The medium was chaged every day and about 2-l0 μl of it was analysed by RIA using the Abbott Ausria II Kit.

## Claims

1. Hepatitis B surface antigen peptide, produced by clone Al26, the amino acid sequence of which corresponds to that of natural HBsAg.

2. A cell line for the production of HBsAg containing the S gene complex which comprises the S gene TATA promoter, the SV40 like promoter and the pre-S$_1$, pre-S$_2$, and S gene coding regions.

3. That cell line of claim 2 additionally containing novel HBV sequences as shown in Fig. I downstream of the 3' end of the S gene.

4. Cell lines according to claims 2 designated CHO$^{50Mr}$ 07.5AL26 and CHO$^{200Mr}$p7.5Al26 and deposited in the Institute Pasteur under accession no. I575 and I574.

5. A method for obtaining a cell line according to claims 2, for the production of HBsAg comprising:
transforming a cell line with an S gene complex from Alexander cells,
and identifying a cell in which the S gene complex is integrated into the host.

6. The method of claim 5 further comprising amplifying the integrated S gene complex using methotrexate.

7. A CHO cell line for the production of HBsAg containing the S gene from hepatoma cell lines.

8. A process for production of HBsAg comprising
culturing the cells of any of claims 2 - I0;
separating the HbsAg from the growth media.

9. A process for production of HBsAg claimed in claim I which comprises:
transforming a cell line with an S gene complex from Alexander Cells;
identifying a colony of cells in which the S gene complex is integrated into the cells;
culturing the cells in a suitable media and separating the HBsAg from the media.

I0. The process of claim 9 wherein the cell line is a Chinese Hamster Ovary (CHO) cell line.

II. The process of claim I0 wherein the S gene complex is contained within the plasmid p7.5Al26.

I2. An HBsAg subunit vaccine comprising an HBsAg protein produced by the processes of claims 8 to II and a pharmaceutically acceptable carrier.

I3. A kit for diagnosing antibodies to Hepatitis B virus containing an antigen defined in claim I, in conjunction with conventional kit components.

I4. A process for the production of a vaccine against human Hepatitis B virus (HBV) which comprises isolating a HBsAg transcriptionally active gene from a heptoma cell line, which produces immunogenic HBsAg 22 nm particles, identifying the individual genes which represent the entire complement of integrated HBV sequence, inserting each of these HBV integrator genes with or without flanking host sequences into a suitable cell line, isolating a clone producing significant quantities of HBsAG, culturing same and recovering the active antigen peptide.

I5. A process according to claim I4, where the hepatoma cells used are Alexander cells.

I6. A process according to claim I4 or I5, where seven distinct genes, representing the entire complement of integrated HBV sequences are introduced into the cells.

I7. A process according to any of claims I4 to I6, where the cells used are CHO dhfr⁻ cells, using dhfr gene as selective marker.

I8. A process according to claims I4, I5 or I7, where the DNA insert is subcloned so as to introduce a 7.5 Kb Eco RI fragment containing the entire integrated HBV sequence and 2 Kb host sequence at the 5' and 3 Kb host sequence at the 3' end of the HBV sequence in a pBT322 plasmid into a CHOfr⁻ cells, testing clones for HBsAg expression and selecting clones having a high expression and using such clone for the production of the active antigen peptide.

I9. A clone designated as 9H, obtained by the process of claim 24, containing the entire coding frames of pre S-I, pre S-2 and S-region, containing the "TATA" box promoter, the SV40 like promoter, the HBV enhancer element and 2-3 polyadenylation sites at the host sequence down the 3' end of the HBV sequence, being devoid of the entire core gene.

20. A vaccine containing as active ingredient HBsAg whenever produced by a cell line obtained according to any of claims I3 to I6.

2I. Anti-HBV vaccines, containing HBsAg obtained from clones defined herein, substantially as hereinbefore described and with reference to any of the Examples.

Figure 1

2536                   1614  1797

$L_J$                      $R_J^*$   $R_J$

HBV

λ_L    RP PS    P    S   XH   R   X     S   S   P X R      R    λ_S

AL 26

10·5
10·7

p7.5

preS2

AL 26    ATGCAGTGGAATTCCACAGCCTTCCACCAAGCTCTGCAACATCC'

                    Ala        His

adyw   ATGCACTGGAACTCCACAACCTTCCACCAAAACTCTGCAAGATCC
ayw    ATGCAGTGGAATTCCACAACCTTCCACCAAAACTCTGCAAGATCC
adw    ATGCAGTGGAATTCCACTGCCTTCCACCAAAACTCTGCAGGATCC

                            1614

                            $R_J^*$

                           GluN

CAGAGTCAGGGGCCTGTATTTTCCTGCTGGTGGCTCCAGTTCAGGAACAGTAAACCCTGCTCAG

                                             Pro

CAGAGTGAGAGGCCTGTATTTCCCTGCTGGTGGCTCCAGTTCAGGGACAGTAAACCCTGTTCCG
CAGAGTGAGAGGCCTGTATTTCCCTGCTGGTGGCTCCAGTTCAGGAACAGTAAACCCTGTTCTG
CAGAGTCAGGGGTCTGTATCTTCCTGCTGGTGGCTCCAGTTCAGGAACAGTAAACCCTGCTCCG

                                Pro

AATATTGCCTCTCACATCTCGTCAATCTCCTCGAUGACTGGGGACCCTGCACCGAACATG

                                Leu

ACTACTACCTCTCCCATATCGTCAATCTTCTCGAGGATTGGGGACCCTGCGCTGAACATG
ACTACTGCCTCTCCCTTATCGTCAATCTTCTCGAGGATTGGGGACCCTGCGCTGAACATG
AATATTGCCTCTCACATCTCGTCAATCTCCGCGAGGACTGGGGACCCTGTGACGAACATG

HBV 1584   CACTTCG CT TCACGTCTGC ACGTCGCATG GAGACCACCG TGAACGGCCA CCA AATAT
            ***** * ** ********** *** ****** **    **    ** * *** *** ** *
AL26 120   CACTTTG CT TCACCTCTGC ACGACGCATG GA    AC    TGG CACCCG CCATGAACA
            *** * ** ****** *** * **    **    *    *****
VHV 1674   G CTTGGTCG TCACCTGTGG A GA    ATT GCGA   ACCA TGGATTCCAC CG TGAACTT

HBV 1641   TGCCCAAGGT CTTACATAAG AGGACTCTTG GACTCTCAGG AATGT CAA CGA CCGAC
            ********** ******** *** * * ***** *** *    ***
AL26 172              CTTACATAAG TGGACTCTTG AACTGT ATT AATGTAGCAA TAATTATCAC
                  * * ** **   *   ****     *    ** * *** *
VHV 1726   TGTCTCCTGG CATGCA AAT CGTCAACTTG GCATG C   C AA G    CAA CGA CCTT

                                            ▼P(A)

HBV 1697   CTTGAGGCAT        ACTT CAA AGACTG TTTGTTTAAA GACTGGGAGG AGTTGGGGCA
            *** * **      *   ** * *    * ** * * * *** ** * *
AL26 221   ATTGTGTAAT GTCGGTTATG TAACA ATCA ATAATTAATG GTTTAGGCAAG AGATTAATGT
            * * * *     *** *** * **** ** *****     * *** ** *
VHV 1775   TGGACTCGT        TATA TAAGAGATCA ATTATTAACT AAATGGGAGG AGGGCAGCAT

                                            ▼P(A)

HBV 1750   GGA GA TTA GGTTAAAGGT GTT TGTACT AGGAGGCTGT AGGCATAAA TTGGTCTGCG
            * *    * ****** * *     * *** ******** *******
AL26 280   TTATGTCCAA GGTTAATGAT AAACTATACA TGGAGGCTGT TAGGCATAGG GATACACCGG
            * * *** * * *** ** * * ** ********* *******     ** *
VHV 1828   TGA   TCCTA GATTATGAAT ATT TGTATT AGGAGGCTGT AGGCATAAA TG CATGCG

                                        ↑
                           $R_J$   1797

0 257 507

## DOCUMENTS CONSIDERED TO BE RELEVANT

EP 87111890.7

| Category | Citation of document with indication, where appropriate, of relevant passages | Relevant to claim | CLASSIFICATION OF THE APPLICATION (Int. Cl. 4) |
|---|---|---|---|
| X | EP - A1 - 0 175 283 (TAKEDA CHEMICAL INDUSTRIES) <br> * Fig. 1 * <br> -- | 1 | C 12 N 15/00 <br> C 12 P 21/00 <br> C 12 N 5/00 <br> A 61 K 39/29 <br> G 01 N 33/576 |
| X | EP - A2 - 0 171 908 (TAKEDA CHEMICAL INDUSTRIES) <br> * Fig. 1,2 * <br> -- | 1 | |
| X | EP - A2 - 0 105 149 (SCIENCE AND TECHNOLOGY AGENCY) <br> * Fig. 2 * <br> -- | 1 | |
| D,X | PROCEEDINGS OF THE NATIONAL ACADEMY OF SCIENCES OF THE UNITED STATES OF AMERICA, vol. 81, no. 24, December 1984, Baltimore, USA <br><br> M.-L. MICHEL et al. "Synthesis in animal cells of hepatitis B surface antigen particles carrying a receptor for polymerized human serum albumin" <br> pages 7708-7712 <br><br> * Abstract * | 7 | **TECHNICAL FIELDS SEARCHED (Int. Cl. 4)** <br><br> C 12 N <br> C 12 P <br> A 61 K <br> G 01 N <br> C 12 Q |
| D,A | * Abstract * <br><br> -- | 9,10 | |
| A | EP - A2 - 0 072 318 (THE REGENTS OF THE UNIVERSITY OF CALIFORNIA) <br> * Claims 1-3,5 * <br> -- | 1,2,5 | |

The present search report has been drawn up for all claims

| Place of search | Date of completion of the search | Examiner |
|---|---|---|
| VIENNA | 25-11-1987 | WOLF |

| | DOCUMENTS CONSIDERED TO BE RELEVANT | | EP 87111890.7 |
| | | | |

| Category | Citation of document with indication, where appropriate, of relevant passages | Relevant to claim | CLASSIFICATION OF THE APPLICATION (Int. Cl.4) |
|---|---|---|---|
| D,A | THE EMBO JOURNAL, vol. 4, no. 1, 1985, Oxford, GB<br><br>A. FREYTAG von LORINGHOVEN et al. "Co-transcribed 3' host sequences augment expression of integrated hepatitis B virus DNA" pages 249-255<br><br>   * Totality *<br><br>-- | 2 | |
| P,A | EP - A1 - 0 198 474 (ENDOTRONICS INC.)<br><br>   * Claims 1,9,17 *<br><br>-- | 2,7 | |
| A | EP - A2 - 0 101 617 (JURIDICAL FOUNDATION THE CHEMO- SERO-THERA-PEUTIC RESEARCH INSTITUTE)<br><br>   * Claims 1,12 *<br><br>-- | 2 | TECHNICAL FIELDS SEARCHED (Int. Cl.4) |
| A | EP - A2 - 0 062 574 (THE REGENTS OF THE UNIVESITY OF CALIFORNIA)<br><br>   * Claims 1-6 *<br><br>-- | 2 | |
| A | WO - A1 - 85/03 876 (GROUPEMENT DE GENIE GENETIQUE et al.)<br><br>   * Abstract; claim 22 *<br><br>-- | 12,21 | |

The present search report has been drawn up for all claims

| Place of search | Date of completion of the search | Examiner |
|---|---|---|
| VIENNA | 25-11-1987 | WOLF |

## DOCUMENTS CONSIDERED TO BE RELEVANT

EP 87111890.7

| Category | Citation of document with indication. where appropriate, of relevant passages | Relevant to claim | CLASSIFICATION OF THE APPLICATION (Int Cl 4) |
|---|---|---|---|
| D,A | NATURE, vol. 317, October 10, 1985, New York, London<br><br>P. TIOLLAIS et al. "The hepatitis B virus"<br>pages 489-495<br><br>   * Totality *<br><br>     ---- | | |
| | | | **TECHNICAL FIELDS SEARCHED (Int. Cl.4)** |

The present search report has been drawn up for all claims

| Place of search | Date of completion of the search | Examiner |
|---|---|---|
| VIENNA | 25-11-1987 | WOLF |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82